(19) Europäisches Patentamt / European Patent Office / Office européen des brevets

(11) **EP 3 981 326 B1**

(12) **EUROPEAN PATENT SPECIFICATION**

(45) Date of publication and mention
of the grant of the patent:
**20.12.2023 Bulletin 2023/51**

(21) Application number: **20865942.5**

(22) Date of filing: **16.09.2020**

(51) International Patent Classification (IPC):
*A61B 5/024* (2006.01)     *A61B 3/113* (2006.01)
*A61B 5/00* (2006.01)

(52) Cooperative Patent Classification (CPC):
**A61B 5/681; A61B 5/02405; A61B 5/02416;
A61B 5/02438; A61B 5/0245; A61B 5/4812;
A61B 5/4815**

(86) International application number:
**PCT/CN2020/115575**

(87) International publication number:
**WO 2021/052362 (25.03.2021 Gazette 2021/12)**

(54) **DATA DISPLAY METHOD AND ELECTRONIC DEVICE**

DATENANZEIGEVERFAHREN UND ELEKTRONISCHE VORRICHTUNG

PROCÉDÉ D'AFFICHAGE DE DONNÉES ET DISPOSITIF ÉLECTRONIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.09.2019 CN 201910876349**

(43) Date of publication of application:
**13.04.2022 Bulletin 2022/15**

(73) Proprietor: **Honor Device Co., Ltd.
Shenzhen, Guangdong 518040 (CN)**

(72) Inventors:
• **ZHANG, Jie**
  **Shenzhen, Guangdong 518040 (CN)**
• **LI, Hongbao**
  **Shenzhen, Guangdong 518040 (CN)**
• **YANG, Bin**
  **Shenzhen, Guangdong 518040 (CN)**
• **LI, Jing**
  **Shenzhen, Guangdong 518040 (CN)**
• **CHEN, Yixin**
  **Shenzhen, Guangdong 518040 (CN)**

(74) Representative: **Gill Jennings & Every LLP
The Broadgate Tower
20 Primrose Street
London EC2A 2ES (GB)**

(56) References cited:
CN-A- 107 157 459      CN-A- 108 185 988
CN-A- 110 037 668      CN-A- 110 623 652
CN-U- 209 346 194      RU-C2- 2 315 551
US-A- 4 796 620        US-A1- 2012 179 061
US-A1- 2017 367 601    US-A1- 2018 070 833
US-A1- 2019 159 676

• TAKAMURA T ET AL: "Effects of bunazosin and propranolol on ventricular arrhythmias in dogs with hypokalemia", JOURNAL OF ELECTROCARDIOLOGY, ELSEVIER SCIENCE, XX, vol. 20, no. 2, 1 January 1987 (1987-01-01), pages 147-153, XP022698258, ISSN: 0022-0736, DOI: 10.1016/S0022-0736(87)80104-8 [retrieved on 1987-01-01]

**Description**

**TECHNICAL FIELD**

This application relates to the field of electronic technologies, and in particular, to a data display method and an electronic device.

**BACKGROUND**

Wearable devices (for example, smart watches and smart bands) can detect physiological data of a human body such as sleep stage data, pressure index data, and blood pressure. The sleep stage data is used as an example. A sleep state of the human body may be controlled by autonomic nerves of the human body, and an autonomic nerve function of the human body may be reflected by pulse data of the human body. Based on this, a wearable device can detect and display the sleep stage data of the human body, for example, by detecting the pulse data of the human body.

However, arrhythmia affects the pulse data, and the corresponding pulse data during arrhythmia is not controlled by an autonomic nervous system. Therefore, if a user has arrhythmia, the sleep stage data detected and displayed by the wearable device is incorrect data.

RU 2 315 551 C2 describes a method for evaluating the effect of prophylactic anti-arrhythmic therapy in patients with frequent paroxysm of atrial fibrillation and flutter according to data of spontaneous relapses of arrhythmia.

**SUMMARY**

This application provides a data display method and an electronic device, which can resolve the problem that displayed data is incorrect data.
According to a first aspect, this application provides a data display method, including: acquiring pulse data within a first time period, a length of the first time period being a first duration; determining that the pulse data includes abnormal pulse data, the abnormal pulse data being pulse data corresponding to arrhythmia; determining a ratio
of a second duration to the first duration, the second duration being an accumulated duration of the abnormal pulse data; and displaying first physiological data when the ratio is greater than a first preset value, the first preset value being a value greater than 0 and less than 1, the first physiological data including a first physiological feature parameter.

After the electronic device determines that the acquired pulse data includes the abnormal pulse data, in order to quantify a relative length of the accumulated duration of the arrhythmia, the ratio of the second duration to the first duration is determined. The first duration is the length of the first time period, and the second duration is the accumulated duration of the abnormal pulse data. When the ratio is greater than the first preset value, it may be considered that the accumulated duration of the arrhythmia is relatively long, and the electronic device may display the first physiological data of a user, the first physiological data including the first physiological feature parameter. In this way, correct physiological data can be displayed when the user has a certain degree of arrhythmia symptoms.

According to the first aspect, the first physiological data and second physiological data are displayed when the ratio is greater than the first preset value and less than or equal to a second preset value, the second preset value being greater than the first preset value and less than 1, the second physiological data including a second physiological feature parameter. With this implementation, the electronic device can display as many physiological feature parameters as possible on the basis of ensuring that correct physiological data is displayed, thereby improving the user experience.

According to the first aspect, third physiological data is displayed when the ratio is less than or equal to the first preset value, the third physiological data including the first physiological feature parameter, a second physiological feature parameter, and a third physiological feature parameter. When the ratio is less than or equal to the first preset value, it indicates that the accumulated duration of arrhythmia of the user is relatively short. Correspondingly, the electronic device can display more physiological feature parameters of the user. This can not only ensure the correctness of the displayed physiological data, but also makes the displayed physiological data more detailed.

In a possible design, before the displaying first physiological data, the method further includes: parsing the pulse data by using an acceleration ACC detection method to obtain the first physiological data. In this application, when there is a particular amount of abnormal pulse data (the ratio is greater than the first preset value) in the pulse data, correct physiological data cannot be obtained by parsing the pulse data by using a cardiopulmonary coupling (cardiopulmonary coupling, CPC) detection method or a heart rate variability (heart rate variability, HRV) detection method. Therefore, the electronic device parses the pulse data by using an acceleration (acceleration, ACC) detection method, thereby obtaining correct physiological data.

In a possible design, before the displaying the first physiological data and second physiological data, the method further includes: parsing the abnormal pulse data in the pulse data by using an acceleration ACC detection method to obtain the first physiological data; and parsing data in the pulse data except the abnormal pulse data by using a cardiopulmonary coupling CPC detection method to obtain the second physiological data. In some embodiments, when the ratio is greater than the first preset value and less than or equal to the second preset value, the electronic device may parse the abnormal pulse data in the pulse data by using the ACC detection method to obtain the first physiological data, and parse the non-abnormal pulse data in the pulse data by using the CPC detection method to obtain the second physiological data, thereby displaying as many physiological feature parameters as possible on the basis of ensuring that correct physiological data is displayed.

In a possible design, before the displaying third physiological data, the method further includes: processing the abnormal pulse data in the pulse data to obtain reconstructed pulse data; and parsing the reconstructed pulse data by using a CPC detection method to obtain the third physiological data. In this application, when there are relatively few abnormal pulse data in the pulse data (the ratio is less than or equal to the first preset value), the electronic device may obtain the third physiological data according to most of the non-abnormal pulse data. Based on this, the electronic device may process the abnormal pulse data in the pulse data to obtain the reconstructed pulse data, where the reconstructed pulse data does not include the abnormal pulse data. Further, the electronic device parses the reconstructed pulse data by using the CPC detection method to obtain the third physiological data, so that correct and detailed physiological data can be obtained.

In a possible design, the processing the abnormal pulse data in the pulse data to obtain reconstructed pulse data includes: deleting the abnormal pulse data from the pulse data to obtain the reconstructed pulse data; or modifying the abnormal pulse data according to non-abnormal pulse data adjacent to the abnormal pulse data, to obtain the reconstructed pulse data. With this implementation, the electronic device can obtain data that does not include the abnormal pulse data according to the pulse data, and can further use the CPC detection method to generate the third physiological data.

In a possible design, the determining that the pulse data includes abnormal pulse data includes: dividing the pulse data into k data segments, k being greater than or equal to 1, a duration corresponding to each data segment in the k data segments being a third duration, the third duration being less than the second duration; and determining whether each data segment in the k data segments satisfies a correspondence $y_t(w \times x_t - b) \geq 1$, t = 1, 2, 3, ..., k, w being a normal vector of support vectors, b being a deviation value of a decision plane, $x_t$ being a $t^{th}$ data segment in the k data segments, $y_t$ being corresponding pulse index data indicating whether heart rhythm is abnormal; and if $y_t$ is pulse index data corresponding to arrhythmia, determining that the data segment is the abnormal pulse data when the data segment satisfies the correspondence $y_t(w \times x_t - b) \geq 1$. With this implementation, the electronic device can determine whether the pulse data includes abnormal pulse data, and further, the electronic device can display different physiological data according to the included abnormal pulse data.

According to the first aspect, the first physiological feature parameter includes a deep sleep duration and a light sleep duration; the second physiological feature parameter includes a rapid eye movement duration; and the third physiological feature parameter includes at least one of a deep sleep ratio, a light sleep ratio, a rapid eye movement ratio, a deep sleep continuity score, a sleep quality score, and a breathing quality score. As can be seen, the electronic device can display different physiological feature parameters according to a relative amount of abnormal pulse data, thereby ensuring the correctness of the displayed physiological data.

According to a second aspect, this application provides an electronic device, comprising: a sensor, a processor, and a display, wherein the sensor is configured to acquire pulse data within a first time period, a length of the first time period being a first duration; the processor is configured to determine that the pulse data comprises abnormal pulse data, the abnormal pulse data being pulse data corresponding to arrhythmia; the processor is further configured to determine a ratio of a second duration to the first duration, the second duration being an accumulated duration of the abnormal pulse data; and the display is configured to display first physiological data when the ratio is greater than a first preset value, the first preset value being a value greater than 0 and less than 1, the first physiological data comprising a first physiological feature parameter; the display is further configured to display the first physiological data and second physiological data when the ratio is greater than the first preset value and less than or equal to a second preset value, the second preset value being greater than the first preset value and less than 1, the second physiological data comprising a second physiological feature parameter; and the display is further configured to display third physiological data when the ratio is less than or equal to the first preset value, the third physiological data comprising the first physiological feature parameter, the second physiological feature parameter, and a third physiological feature parameter, wherein the first physiological feature parameter comprises a deep sleep duration and a light sleep duration; the second physiological feature parameter comprises a rapid eye movement duration; and the third physiological feature parameter comprises at least one of a deep sleep ratio, a light sleep ratio, a rapid eye movement ratio, a deep sleep continuity score, a sleep quality score, and a breathing quality score.

According to a third aspect, this application provides a computer storage medium. The computer storage medium stores instructions, the instructions, when run on a computer, causing the computer to perform part or all of the steps of the data display method in the first aspect and various possible implementations of the first aspect.

To resolve the problems in the prior art, in the technical solution of this application, after determining that acquired pulse data includes abnormal pulse data, the electronic device calculates a ratio of an accumulated duration of the abnormal pulse data to a total duration of the pulse data, and further determines whether the ratio is greater than a first preset value. When the ratio is greater than the first preset value, the electronic device displays first physiological data of a user, where the first physiological data includes a first physiological feature parameter, thereby ensuring that the physiological data displayed to the user is correct, and improving the user experience.

**BRIEF DESCRIPTION OF DRAWINGS**

FIG. 1A is an exemplary schematic structural diagram of a smart watch 10 according to this application;

FIG. 1B is an exemplary schematic structural diagram of an electronic device 100 according to this application;

FIG. 1C is an exemplary schematic diagram of a display screen in FIG. 1A according to this application;

FIG. 2A is a schematic diagram of a first implementation of a user interface according to this application;

FIG. 2B is a schematic diagram of a second implementation of a user interface according to this application;

FIG. 2C is a schematic diagram of a third implementation of a user interface according to this application;

FIG. 2D is a schematic diagram of a fourth implementation of a user interface according to this application;

FIG. 2E is a schematic diagram of a fifth implementation of a user interface according to this application;

FIG. 3 is an exemplary method flowchart of a data display method 10 according to this application;

FIG. 4A is an exemplary schematic structural diagram of an electronic device 40 according to this application; and

FIG. 4B is an exemplary schematic structural diagram of an electronic device 41 according to this application.

**DESCRIPTION OF EMBODIMENTS**

The following clearly describes technical solutions of this application with reference to the accompanying drawings in this application.

Terms used in the following embodiments of this application are only intended to describe particular embodiments, and are not intended to limit this application. As used in this specification and the claims of this application, a singular expression form, "one", "a", "said", "foregoing", "the", or "this", is intended to also include a plural expression form, unless clearly indicated to the contrary in the context. It should be understood that although the terms first, second, etc. may be used in the following embodiments to describe a certain type of object, the object should not be limited to the terms. The terms are only used to distinguish specific objects of this type of objects. For example, in the following embodiments, the terms first, second, etc. may be used to describe the duration, but the duration should not be limited to the terms. The terms are only used to distinguish between different lengths of time. In the following embodiments, the terms first, second, etc. may be used to describe other types of objects in the same way, and the details are not repeated herein. In addition, the term "and/or" used in this application indicates and includes any or all possible combinations of one or more listed items.

The following embodiments introduce an electronic device and an embodiment of a data display method related to the electronic device.

In some embodiments, the electronic device described in this application may be a wearable device, and the wearable device may be any device with a function of detecting physiological data of a user. The wearable device may be, for example, a smart watch, a smart band, or a bracelet that is supported by a wrist, or may be, for example, a smart shoe or a sock that is supported by a foot, or another product worn on a leg in the future, or may be smart glasses, a smart helmet, and a headband supported by a head. In some other embodiments, the electronic device described in this application may be another device configured to detect a physiological parameter of the user. For example, the electronic device is a device that uses radar waves to detect pulse waves of the user. In another example, the electronic device is a device that uses a mattress to acquire a ballistocardiogram signal of the user. In another example, the electronic device is a device that detects pulse waves by using a camera of a mobile phone to take pictures of bare skin of the user. This is not limited in this application.

This application relates to a "user interface (user interface, UI)" configured to display data. The UI is a medium interface for interaction and information exchange between an application or operating system and a user, and implements the conversion between an internal form of information and a form of the information acceptable to the user. The user interface of the application is source code written in a specific computer language such as java and the extensible markup language (extensible markup language, XML). The interface source code is parsed and rendered on a terminal device, and is finally presented as content that can be recognized by the user, such as a picture, a text, a button and other controls. A control (control), also referred to as a widget (widget), is a basic element of the user interface. Typical controls include a toolbar (toolbar), a menu bar (menu bar), a text box (text box), a button (button), a scrollbar (scrollbar), a picture, and a text. The attributes and content of the controls in the interface are defined by tags or nodes. For example, XML specifies the controls included in the interface through nodes such as <Textview>, <ImgView>, and <VideoView>. One node corresponds to one control or attribute in the interface, and the node is parsed and rendered, and is then presented as user-visible content. In addition, interfaces of many applications, such as hybrid applications (hybrid application), usually further include web pages. A web page, also referred to as a page, may be understood as a special control embedded in an application interface. The web page is source code written in a specific computer language, such as the HyperText Markup Language (HyperText Markup Language, HTML), cascading style sheets (cascading style sheets, CSS), and java scripts (JavaScript, JS). The source code of the web page may be loaded and displayed by a browser or a web page display component with similar functions to the browser as content that can be recognized by the user. The specific content included in the web page is also defined by tags or nodes in the source code of the web page. For example, HTML defines elements and attributes of the web page through <p>, <img>, <video>, and <canvas>.

Some of physiological functions of a human body such as sleep and breathing are controlled by autonomic nerves, and the role of the autonomic nerves may be reflected in the heart rhythm of the human body. Generally, a blood volume in blood vessels increases under the action of heart contraction, and the blood volume in the blood vessels decreases under the action of heart relaxation. Based on this, the heart rhythm of the human body may be reflected in the pulse of the human body. Further, in actual operation, the electronic device may detect physiological data of the user such as blood pressure, blood oxygen, brain oxygen, muscle oxygen, blood sugar, pulse rate, and respiration rate by acquiring and detecting pulse data (also referred to as pulse wave data or photo plethysmo graphy (photo plethysmo graphy, PPG) data) of the user. However, arrhythmia (such as atrial fibrillation) is not controlled by an autonomic nervous system. If the user has symptoms of arrhythmia, the pulse data acquired by the electronic device includes pulse data corresponding to the arrhythmia, and consequently, the physiological data of the user parsed and displayed by the electronic device is incorrect.

This application provides a data display method and an electronic device. Even if a user using the electronic device has symptoms of arrhythmia, the electronic device can still parse and display correct physiological data of the user according to acquired pulse data, thereby improving the user experience.

The following describes an exemplary electronic device 100 involved in an embodiment of this application.

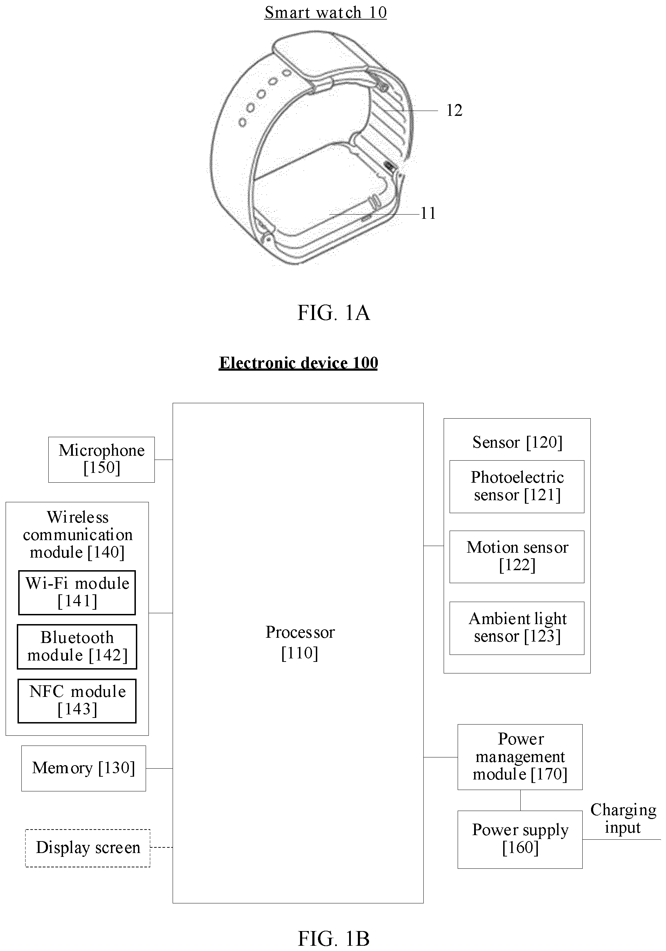

The electronic device 100 involved in this application may be, for example, a smart watch 10 shown in FIG. 1A, and the smart watch 10 includes a watch body 11 and a bracelet 12. The bracelet 12 is configured to enable the user to wear the smart watch 10, and the watch body 11 is configured to implement some or all of the embodiments of the data display method involved in this application.

It may be understood that the smart watch 10 shown in FIG. 1A constitutes no specific limitation on the electronic device 100. In some other embodiments of this application, the electronic device 100 may be a smart band, smart glasses, a smart helmet, or the like, and the details are not described herein again.

FIG. 1B is a schematic structural diagram of the electronic device 100. The devices shown in FIG. 1B may be disposed in the watch body 11 of the smart watch 10 shown in FIG. 1A.

The electronic device 100 may include a processor 110, a sensor 120, a memory 130, a wireless communication module 140, a microphone (microphone, MIC) 150, a power supply 160, a power management module 170, and the like. The wireless communication module 140 may include a wireless fidelity (wireless fidelity, Wi-Fi) module 141, a Bluetooth module 142, a near field communication (near field communication, NFC) module 143, and the like. The sensor 120 may include a photoelectric sensor 121 for detecting physiological signals of a human body, a motion sensor 122, and an ambient light sensor 123.

It may be understood that the schematic structure in this application constitutes no specific limitation on the electronic device 100. In some other embodiments of this application, the electronic device 100 may include more or fewer components than those shown in the figure (for example, a display screen and a gyroscope), or some components may be combined, or some components may be split, or components are arranged in different manners. The components shown in the figure may be implemented by hardware, software, or a combination of software and hardware.

The processor 110 is configured to perform system scheduling, such as calling the Wi-Fi module 141, the Bluetooth module 142, and the NFC module 143 to support the processing of the microphone 150, and the like. The processor 110 is further configured to: detect, according to data inputted by the sensor 120, whether the user has arrhythmia, and then use corresponding detection methods for different scenarios to detect physiological data of the user.

The photoelectric sensor 121 for detecting physiological signals of the human body may be, for example, a PPG sensor or an electrocardiogram (electrocardiogram, ECG) sensor. The photoelectric sensor 121 for detecting physiological signals of the human body may be configured on a side of the electronic device 100 that is in contact with skin of the user when the electronic device is worn. The photoelectric sensor 121 for detecting physiological signals of the human body may be configured to, for example, detect changes in light intensity, and convert the detected changes in light intensity into digital electrical signals, such as PPG signals. The photoelectric sensor 121 for detecting physiological signals of the human body may be further configured to detect electrocardiographic signals, vibration signals, bio-impedance signals, and the like of the human body. Further, the photoelectric sensor 121 for detecting physiological signals of the human body is further configured to send the detected signals, such as the PPG signals, the electrocardiogram signals, the vibration signals, and the bio-impedance signals to the processor 110, so that the processor 110 obtains pulse data of the user from the corresponding signals, and further measures the physiological data of the user according to the obtained pulse data.

The motion sensor 122 may be, for example, an acceleration sensor. The motion sensor 122 is configured to detect an acceleration value of the electronic device 100 in each direction (generally three axes).

The ambient light sensor 123 is configured to detect intensity of ambient light and convert the intensity of the ambient light into a data electrical signal.

With reference to FIG. 1A, the PPG sensor or the ECG sensor may be disposed on the inner side of the watch body 11. After the user wears the smart watch 10, the PPG sensor or the ECG sensor may be in contact with the body of the user. The inner side of the watch body 11 is the side in contact with the body of the user when the user wears the smart watch 10.

The memory 130 is configured to store a software program and data. The processor 110 implements the embodiments of the data display method of this application by running the software program. The data is used for providing support for the processor 110 to run the software program. The memory 130 may include a program storage area and a data storage area. The program storage area may store an operating system, and an application required by at least one function (such as a data display function and a sound playback function). The data storage area may store data created according to use of the electronic device 100, an algorithm required for the processor 110 to perform physiological data detection, and the like. In addition, the memory may include a high-speed random access memory, and may further include a non-volatile memory, for example, a magnetic disk storage device, a flash memory device, or another volatile solid-state storage device.

The wireless communication module 140 may provide a solution to wireless communication applied to the electronic device 100, for example, a wireless local area network (wireless local area networks, WLAN) (for example, a Wi-Fi network), Bluetooth (Bluetooth, BT), a global navigation satellite system (global navigation satellite system, GNSS), NFC, and an infrared (infrared, IR) technology. The wireless communication module 140 may be one or more components into which at least one communication processing module is integrated. The wireless communication module 140 receives an electromagnetic wave through an antenna, performs frequency modulation and filtering processing on the electromagnetic wave signal, and sends the processed signal to the processor 110. The wireless communication module 140 may further receive a to-be-sent signal from the processor 110, perform frequency modulation and amplification on the to-be-sent signal, and convert the signal into an electromagnetic wave through an antenna and radiate the signal. In some embodiments, the solution to wireless communication provided by the wireless communication module 140 can enable the electronic device 100 to exchange data with other electronic devices (such as mobile phones and tablet computers). For example, the physiological data of the user may be displayed through other electronic devices.

The microphone 150, also referred to as a mike, may convert acquired sound signals into electrical signals, and the electrical signals are received by an audio circuit and converted into audio data. The audio circuit may also convert audio data into electrical signals, and the electrical signals are transmitted to a speaker, and converted into sound signals by the speaker for output.

The power management module 170 is configured to connect the power supply 160 and the processor 110. The power management module 170 receives input of the power supply 160 and supplies power to the processor 110, the sensor 120, the memory 130, the wireless communication module 140, the microphone 150, and other components. The power management module 170 may be further configured to monitor parameters of the power supply 160 such as a capacity, a cycle count, and a state of health (electric leakage and impedance).

The electronic device 100 may include a display screen, and the display screen is configured to display controls, data, images, and the like. The display screen includes a display panel. The display panel may be a liquid crystal display (liquid crystal display, LCD), an organic light-emitting diode (organic light-emitting diode, OLED), an active-matrix organic light emitting diode (active-matrix organic light emitting diode, AMOLED), a flexible light-emitting diode (flex light-emitting diode, FLED), a Miniled, a MicroLed, a Micro-oLed, quantum dot light emitting diodes (quantum dot light emitting diodes, QLED), and the like.

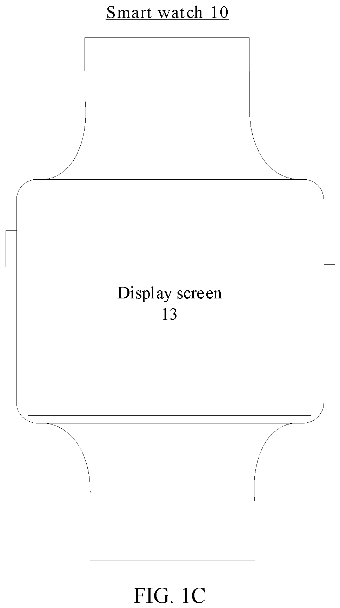

Exemplarily, the display screen 13 of the smart watch 10 may be shown in FIG. 1C. In some embodiments, the smart watch 10 may display physiological data involved in this application on the display screen 13.

The electronic device 100 exemplarily shown in FIG. 1A to FIG. 1C may obtain pulse data of a user through the sensor 120, and the processor 110 then parses the pulse data to obtain physiological data of the user. Further, in some embodiments, the display screen of the electronic device 100 displays various user interfaces described in the following embodiments. In some other embodiments, the processor 110 calls the wireless communication module 140 to send the physiological data of the user to another electronic device, to display various user interfaces described in the following embodiments through the another electronic device.

The following describes exemplary user interfaces in different scenarios of this application.

In this application, if the user has no symptom of arrhythmia, or the user has symptoms of arrhythmia but an accumulated duration of the arrhythmia is relatively short, the electronic device 100 may display more physiological data of the user. If the user has symptoms of arrhythmia and an accumulated duration of the arrhythmia is relatively long, the electronic device 100 may display fewer physiological data of the user.

Exemplarily, to quantify a relative length of the accumulated duration of the arrhythmia, this application may use a ratio of the accumulated duration of the arrhythmia to a total duration of acquiring the pulse data as the basis for determining the accumulated duration of the arrhythmia. Based on this, "the accumulated duration of the arrhythmia is relatively short" indicates, for example, that the corresponding ratio is less than or equal to a first preset value, and "the accumulated duration of the arrhythmia is relatively long" indicates, for example, that the corresponding ratio is greater than the first preset value. The first preset value is a value greater than 0 and less than 1, for example, 5%. This is not limited in this application.

In addition, for ease of description, in this application, the physiological data of the user is summarized into first physiological data, second physiological data, and third physiological data. The first physiological data includes a first physiological feature parameter; the second physiological data includes a second physiological feature parameter; and the third physiological data includes the first physiological feature parameter, a second physiological feature parameter, and a third physiological feature parameter.

Exemplarily, display of sleep stage data of the user on a smartphone is used as an example below to introduce the user interface involved in this application. After the electronic device 100 determines the sleep stage data of the user, the sleep stage data is, for example, sent to a smartphone, and the smartphone then presents the following user interface on a display screen of the smartphone.

The sleep of the user may include two states: non-rapid eye movement sleep (non-rapid eye movement sleep, NREM sleep) and rapid eye movement sleep (rapid eye movement sleep, REM sleep). The NREM sleep stage may further include four periods. For example, the first period is a falling asleep stage, the second period is a light sleep stage, the third period is a moderate sleep stage, and the fourth period is a deep sleep stage.

The sleep stage data is used as an example. The first physiological feature parameter includes, for example, a deep sleep duration and a light sleep duration. The second physiological feature parameter includes, for example, a rapid eye movement duration. The third physiological feature parameter includes, for example, at least one of a deep sleep ratio, a light sleep ratio, a rapid eye movement ratio, a deep sleep continuity score, a sleep quality score, and a breathing quality score. Correspondingly, the first physiological data includes the deep sleep duration and the light sleep duration; the second physiological data includes the rapid eye movement duration; and the third physiological data includes at least one of the deep sleep ratio, the light sleep ratio, the rapid eye movement ratio, the deep sleep continuity score, the sleep quality score, and the breathing quality score.

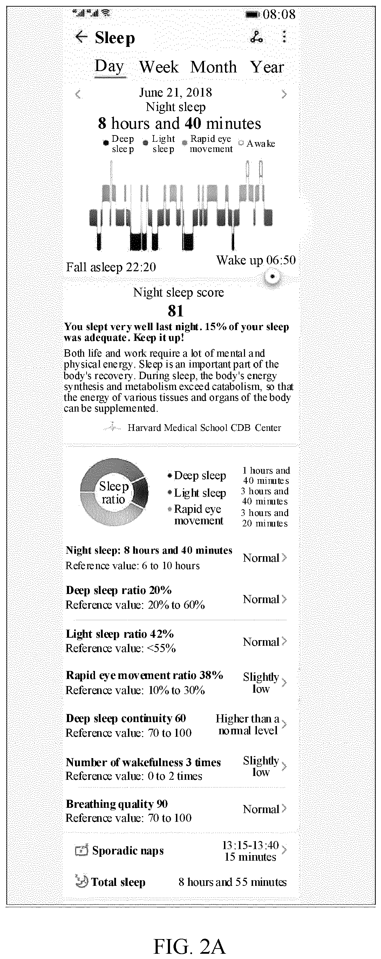

The exemplary user interface shown in FIG. 2A presents the third physiological data. The current date is June 21, 2018. The user fell asleep at 22:20 on June 20, 2018 and woke up at 6:50 on June 21, 2018. A sleep duration during the night is 8 hours and 40 minutes. A curve of sleep stages in time periods during the sleep is presented. In this embodiment, the third physiological data further includes: a night sleep score of the user calculated by the electronic device 100 being 81 with an evaluation attached, a deep sleep duration of the user being 1 hour and 40 minutes, a light sleep duration being 3 hours and 40 minutes, a rapid eye movement duration being 3 hours and 20 minutes, a proportion of a total duration of each sleep stage in the total sleep duration, a normal range of the total duration of each sleep stage, and analysis results. For example, a proportion of deep sleep being 20% is normal, and a proportion of rapid eye movement being 38% is slightly low. The third physiological data may even include time periods and durations of sporadic naps the day before the current date of the user, and a total sleep duration of the sporadic naps during the day and the night sleep. The details are not described herein in this application.

It should be pointed out that the user interface shown in FIG. 2A presents the physiological data when the user has no arrhythmia symptoms. If the user has symptoms of arrhythmia and the corresponding ratio is less than or equal to the first preset value, the user interface may further display warning information in addition to the third physiological data. For example, a start moment corresponding to sleep data acquired by the electronic device 100 is 22:30 on June 20, 2018, an end moment is 7:00 on June 21, 2018, and a total duration of the acquired sleep data is 8 hours and 30 minutes. A total duration of night sleep is, for example, 8 hours and 20 minutes (including deep sleep, light sleep, and rapid eye movement), and a total duration of arrhythmia is, for example, 10 minutes. In this scenario, the user interface may further present warning information such as "the user has slight arrhythmia". Other data in the user interface in this scenario is similar to that in FIG. 2A, and the details are not repeated herein.

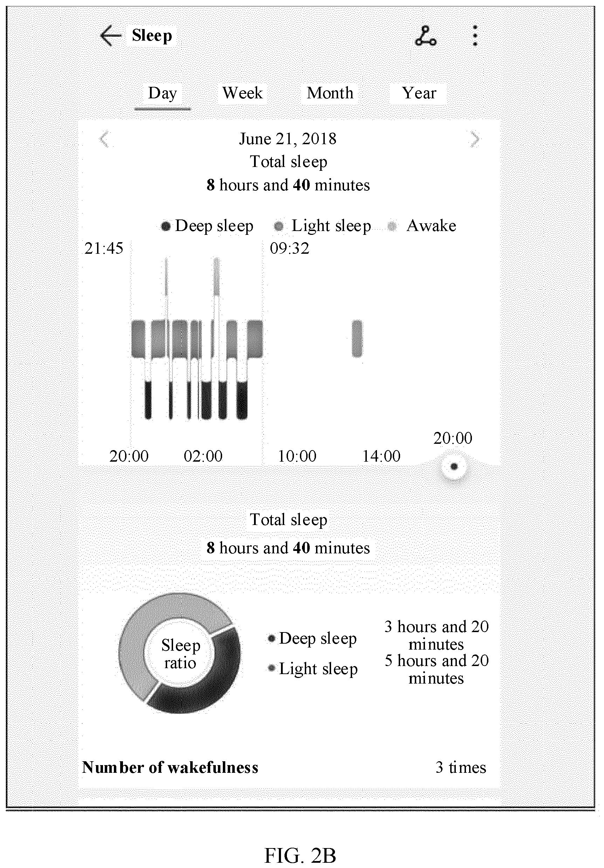

The exemplary user interface shown in FIG. 2B presents the first physiological data. A current date is June 21, 2018. The user fell asleep at 20:00 on June 20, 2018 and woke up at 9:32 on June 21, 2018. A sleep duration during the night is 8 hours and 40 minutes. A total duration of arrhythmia is 2 hours. A stage curve of deep sleep, light sleep, and wakefulness in each period is shown in FIG. 2B. A deep sleep duration is 3 hours and 20 minutes. A light sleep duration is 5 hours and 20 minutes.

As can be seen, when the user has no symptoms of arrhythmia or has symptoms of arrhythmia for a relatively short duration, the electronic device 100 can display the first physiological feature parameter, the second physiological feature parameter, and the third physiological feature parameter of the user. When the user has symptoms of arrhythmia for a relatively long duration, the electronic device 100 only displays the first physiological feature parameter of the user, thereby ensuring that the displayed physiological data is correct.

In some other embodiments, when the ratio is greater than the first preset value and less than the second preset value, the electronic device may further display the first physiological data and the second physiological data of the user. The second preset value is greater than the first preset value and less than 1, and the second preset value is, for example, 30%.

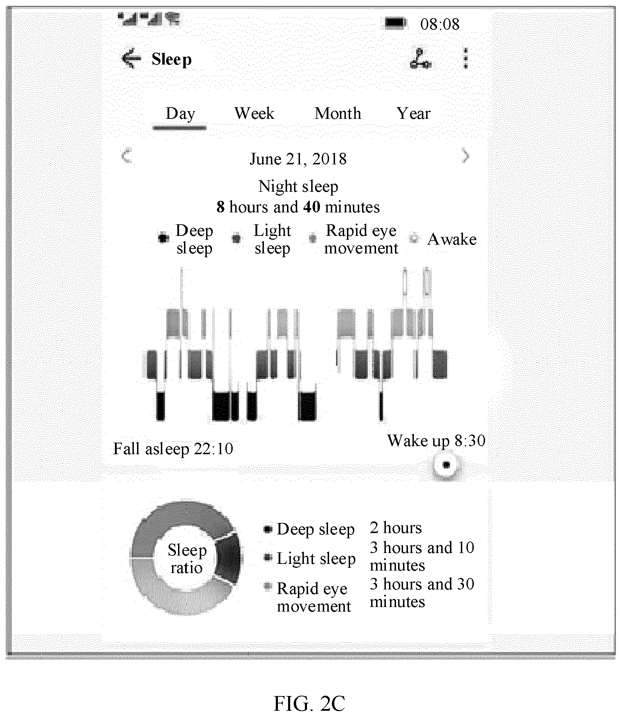

The exemplary user interface shown in FIG. 2C presents the first physiological data and the second physiological data. The current date is June 21, 2018. The user fell asleep at 22: 10 on June 20, 2018 and woke up at 8:30 on June 21, 2018. A sleep duration during the night is 8 hours and 40 minutes. The first physiological data includes: a deep sleep duration being 2 hours and a light sleep duration being 3 hours and 10 minutes. The second physiological data includes: a rapid eye movement duration being 3 hours and 30 minutes.

With this implementation, the electronic device 100 can display as many physiological feature parameters as possible on the basis of ensuring that correct physiological data is displayed, thereby improving the user experience.

It may be understood that "June 21, 2018" displayed on the user interfaces shown in FIG. 2A to FIG. 2C are only a schematic description, and does not constitute a limitation to the technical solution of this application. In actual use, when the user triggers the arrow on the left or right side of "June 21, 2018", sleep stage data before or after "June 21, 2018" may be displayed on the user interface. For example, when the user clicks on the arrow on the left of "June 21, 2018", the sleep stage data on June 20, 2018 may be displayed on the user interface. In another example, if "June 21, 2018" is not the current time, and the user clicks on the arrow on the right side of "June 21, 2018", the sleep stage data on June 22, 2018 may be displayed on the user interface.

Further, in some embodiments, the electronic device 100 may further generate and display heart rhythm statistics of the user. Exemplarily, with reference to the user interfaces shown in FIG. 2A to FIG. 2C, the user may view heart rhythm statistics for one day by triggering "Day" on the user interface, and may further view heart rhythm statistics for a week by triggering "Week" on the user interface. The heart rhythm statistics may include, for example, whether the heart rhythm is abnormal, a change curve of the heart rhythm, the irregular heart rhythm, and the time when the irregular heart rhythm occurs.

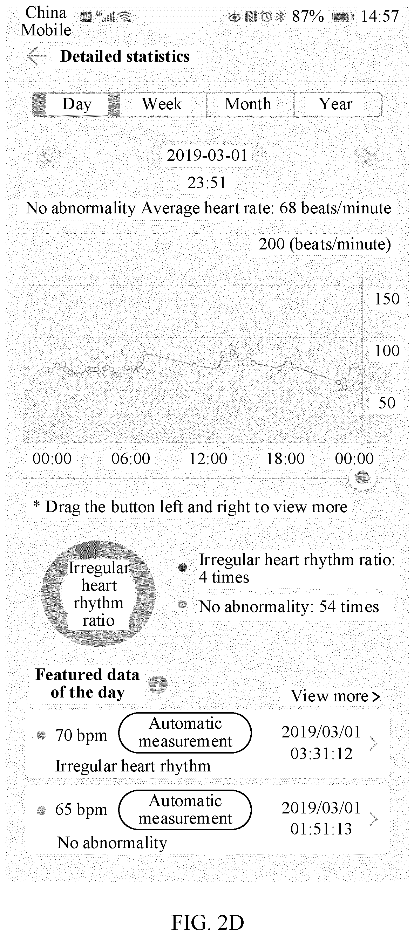

FIG. 2D shows a user interface of exemplary heart rhythm statistics. The heart rhythm statistics are, for example, heart rhythm statistics of the user on March 1, 2019. The heart rhythm statistics include data such as a statistical result "No abnormality", "Average heart rhythm: 68 beats/minute", a heart rhythm change curve of the user on March 1, 2019, "Irregular heart rhythm: 4 times", "No abnormality: 54 times", "Irregular heart rhythm is 70 bmp. The time of occurrence is 03:31:12 on March 1, 2019", and "Non-abnormal heart rhythm is 65 bmp". The details are not described in this application.

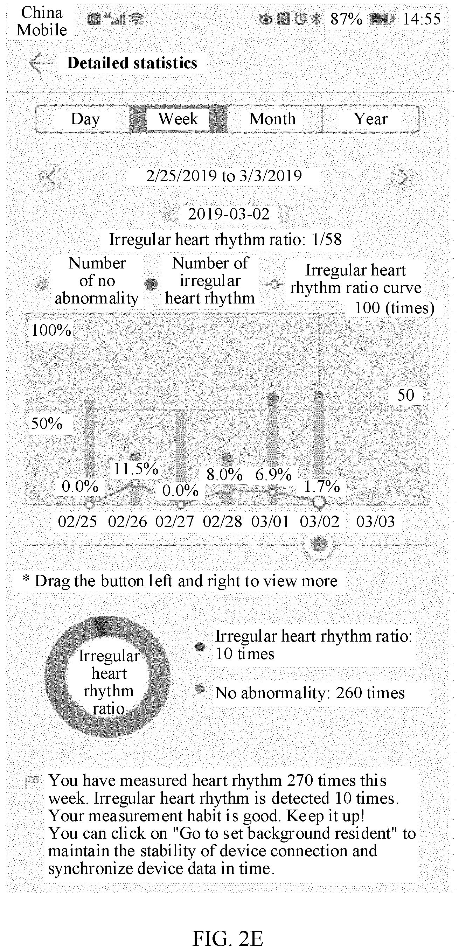

FIG. 2E shows a user interface of exemplary heart rhythm statistics. The heart rhythm statistics are, for example, heart rhythm statistics for the week from February 25, 2019 to March 3, 2019. The heart rhythm statistics for the week include data such as "Irregular heart rhythm ratio: 1/58", "Histogram and graph of the heart rhythm for each day of the week", "Irregular heart rhythm: 10 times", and "No abnormality: 260 times". The details are not described in this application.

As can be seen, with this implementation, the electronic device 100 may further count and display the heart rhythm of the user at various stages, thereby reminding the user of the health status at any time, and improving the user experience.

It may be understood that FIG. 2A to FIG. 2E are only schematic descriptions, and do not constitute a limitation to the protection scope of the embodiments of this application. In some other embodiments, the data presented in the user interface may be alternatively other physiological data of the user, and the expression form of the data presented in the user interface may be alternatively other forms, and the details are not described herein again.

In addition, the user interfaces shown in FIG. 2A to FIG. 2E are user interfaces using a smartphone as an example. In some other embodiments, the electronic device 100 may further display the physiological data on the display screen of the electronic device 100. It should be understood that areas of display screens of different devices may be different, so that quantities of data displayed on user interfaces of different electronic devices are different, which is not limited in this application.

The data display method of this application is exemplarily described below from the perspective of the electronic device 100.

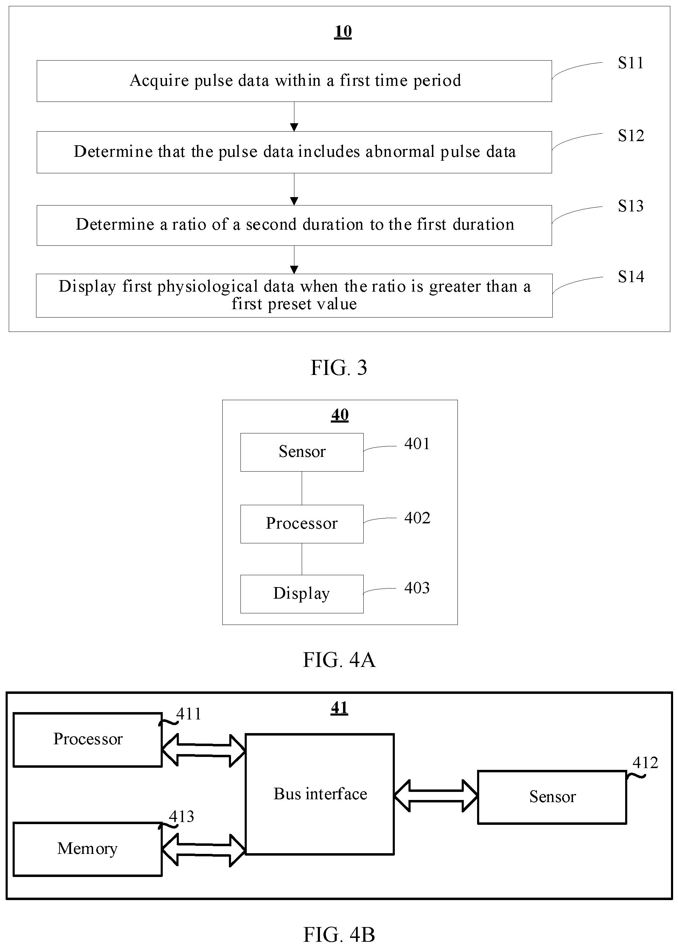

FIG. 3 shows a data display method 10. The data display method 10 (referred to as the method 10 below) includes the following steps:

Step S11: Acquire pulse data within a first time period.

The first time period may be any time period of a day, for example, from 11 o'clock to 13 o'clock in a day, or from 22 o'clock in a day to 7 o'clock in a next day. A length of the first time period is a first duration. Exemplarily, the shortest first duration may be, for example, 1 minute, and the longest first duration may be, for example, 24 hours, which is not limited in this application.

Exemplarily, the pulse data is, for example, PPG data. With reference to the embodiment shown in FIG. 1B, the sensor 121 for detecting physiological signals of the human body is, for example, a photoelectric sensor, and the photoelectric sensor may acquire the pulse data of the user. In some embodiments, the photoelectric sensor is, for example, a PPG sensor. For example, when the heart of the user contracts, the blood volume of the blood vessels increases, the amount of light absorption increases correspondingly, and correspondingly, the light intensity detected by the PPG sensor is relatively low; and when the heart of the user relaxes, the blood volume of the blood vessels decreases, and the light intensity detected by the PPG sensor is relatively high. Based on this, as the heart of the user beats, the PPG sensor may detect the light intensity with the pulsating change, and further, the PPG sensor may convert the light intensity change signal into a digital electrical signal. The digital electrical signal may be referred to as a PPG signal or PPG data, that is, the pulse data described in this solution.

It may be understood that acquiring the pulse data by using the PPG sensor is only a schematic description, and does not constitute a limitation to the technical solutions of the embodiments of this application. In some other embodiments, the electronic device 100 may alternatively acquire the pulse data through an ECG sensor. The details are not described herein in this application.

Step S12: Determine that the pulse data includes abnormal pulse data.

The abnormal pulse data is pulse data corresponding to arrhythmia.

After acquiring the pulse data, the photoelectric sensor transmits the pulse data to the processor 110. The processor 110 detects whether there is arrhythmia pulse data in the pulse data, and if there is arrhythmia pulse data in the pulse data, the processor 110 may count an accumulated duration of arrhythmia of the user to obtain a second duration. Exemplarily, the processor 110 may preset an arrhythmia preliminary screening algorithm, and then use the arrhythmia preliminary screening algorithm to detect whether there is abnormal pulse data in the pulse data. The arrhythmia preliminary screening algorithm is any algorithm that can screen pulse data for arrhythmia, such as a support vector machine (support vector machine, SVM) algorithm, a threshold method, a machine learning method, and a deep learning method. This is not limited in this application.

Exemplarily, the processor 110 divides the PPG data within the first time period into k data segments, where k is greater than or equal to 1, a duration corresponding to each data segment is a third duration, the third duration is less than the second duration, and the third duration is, for example, one minute. The processor 110 then uses the SVM algorithm to separately detect whether each data segment is abnormal data.

The SVM algorithm determines a detection result by calculating a distance between each data segment and a decision plane in a feature space. For example, $y_t(w \times x_t - b) \geq 1$ is a condition correspondence that each data segment should meet, and

$$\arg \min_{(w,b)} \frac{1}{2} \|w\|^2$$ defines values of w and b when $y_t(w \times x_t - b) \geq 1$ reaches a minimum value. w refers to a normal vector of support vectors (formed by vectors closest to the decision plane), $w = \sum_{t=1}^{k} a_t y_t x_t$. b refers to a deviation value of the decision plane (in the feature space, the plane divides samples into two parts, which correspond to arrhythmia samples and normal samples respectively), $b = \frac{1}{m}\sum_{t=1}^{m}(w \times x_t - y_t)$. $x_t$ refers to a $t^{th}$ data segment in the k data segments, such as a heart rhythm variability feature and an entropy feature. $y_t$ refers to pulse index data indicating whether the heart rhythm is abnormal. In some embodiments, when detecting whether a data segment is abnormal pulse data, $y_t$ is index data corresponding to arrhythmia; in some other embodiments, when detecting whether a data segment is normal pulse data, $y_t$ is index data corresponding to normal heart rhythm. $y_t$ may be data in the medical field, t = 1, 2, 3, ..., k refers to a sequence number of each data segment in the k data segments, $a_t$ is the Lagrangian multiplier, and m represents a quantity of the support vectors (formed by the vectors closest to the decision plane). Exemplarily, if $y_t$ is pulse index data corresponding to arrhythmia, it is determined that the data segment $x_t$ is abnormal pulse data when the data segment $x_t$ satisfies the correspondence $y_t(w \times x_t - b) \geq 1$. Similarly, if $y_t$ is pulse index data corresponding to normal heart rhythm, it is determined that the data segment $x_t$ is normal pulse data when the data segment $x_t$ satisfies the correspondence $y_t(w \times x_t - b) > 1$.

Further, after determining the abnormal pulse data in the pulse data, the processor 110 may count an accumulated duration of the abnormal pulse data to obtain a second duration. Further, the processor 110 may determine whether a ratio of the second duration to the first duration is greater than a preset value, and then perform the following steps according to a determination result.

Step S13: Determine a ratio of a second duration to the first duration.

Step S14: Display first physiological data when the ratio is greater than a first preset value.

The first preset value is a value greater than 0 and less than 1, such as 5%, or may be expressed as 0.05. The first physiological data includes a first physiological feature parameter, exemplarily, as described in the embodiment corresponding to FIG. 2B, and the details are not described herein again.

In some embodiments, when the ratio shown in step S13 is greater than the first preset value, the processor 110 may parse the pulse data by using an acceleration (acceleration, ACC) detection method, to obtain the first physiological data of the user. As can be seen, using this implementation method can ensure the accuracy of the obtained physiological data.

In some other embodiments, when the ratio shown in step S13 is greater than the first preset value and less than or equal to the second preset value, in order to present the physiological data of the user as much as possible, the processor 110 may parse abnormal pulse data in the pulse data by using the ACC detection method, to obtain first physiological data; and parse data except the abnormal pulse data in the pulse data by using a cardiopulmonary coupling (cardiopulmonary coupling, CPC) detection method or a heart rate variability (heart rate variability, HRV) detection method, to obtain second physiological data, where the second physiological data includes a second physiological feature parameter. The first physiological data and the second physiological data are then displayed on a display. The second preset value is greater than the first preset value and less than 1, and the second preset value is, for example, 30%, or may be expressed as 0.3. The display examples of the first physiological data and the second physiological data are, for example, as described in the embodiment corresponding to FIG. 2C, and the details are not described herein again.

In still some other embodiments, when the ratio shown in step S13 is less than or equal to the first preset value, the processor 110 may process the pulse data to obtain reconstructed pulse data, and then generate third physiological data, where the third physiological data includes the first physiological feature parameter, a second physiological feature parameter, and a third physiological feature parameter. For the display example of the third physiological data, reference may be made to the embodiment corresponding to FIG. 2A, and the details are not described herein again.

In some embodiments, the processor 110 may delete the abnormal pulse data from the pulse data to obtain the reconstructed pulse data. In some other embodiments, the processor 110 may modify the corresponding abnormal pulse data according to non-abnormal pulse data adjacent to the abnormal pulse data, to obtain the reconstructed pulse data.

The CPC detection method is used as an example below to exemplarily describe the implementation process of obtaining the third physiological data by the electronic device 100.

In this embodiment, the pulse data described in step S11 is PPG data. Based on this, the specific process for the processor to generate the third physiological data includes the following steps:

Step 1: The processor 110 may extract an R peak to R peak interval (R peak to R peak interval, RR) sequence from the PPG data, and then the processor 110 may extract a respiratory signal sequence from the RR sequence.

An algorithm that extracts the respiratory signal sequence from the RR sequence is referred to as an ECG-derived respiratory (ECG-derived respiratory, EDR) algorithm. Therefore, the respiratory signal sequence is also referred to as an EDR signal sequence.

Step 2: The processor 110 uses a sliding window to delete abnormal sample points in the RR sequence to obtain a reconstructed RR sequence.

Step 3. The processor 110 uses an empirical mode decomposition algorithm to decompose the reconstructed RR sequence and the EDR signal sequence, and calculates a coherence and cross power spectrum of the decomposed RR sequence and the decomposed EDR signal sequence.

Step 4. The processor 110 calculates the third physiological data according to a coherent and cross power spectrum result.

The sequences and algorithms involved in the foregoing steps 1 to 4 are all technologies well-known to those skilled in the art, and the details are not described herein again.

In addition, if the pulse data includes abnormal pulse data, in the process of generating the physiological data, the processor 110 may further obtain heart rhythm statistics of the user in different time periods according to statistics of the heart rhythm data of the user. The heart rhythm statistics are described in the embodiment shown in FIG. 2D or FIG. 2E.

Further, with reference to the embodiments shown in FIG. 1A to FIG. 1C, the processor 110 executes a display function and displays physiological data involved in the method 10 through the display screen 13, to obtain data of the user interface shown in any one of FIG. 2A to FIG. 2E. In some other embodiments, the processor 110 may send the physiological data involved in the method 10 to another electronic device through the wireless communication module 140, and the another electronic device may display the corresponding physiological data to obtain the user interface shown in any one of FIG. 2A to FIG. 2E.

It may be understood that the foregoing CPC detection method, ACC detection method, sleep stage data, and the like are all schematic descriptions, and do not constitute a limitation to the technical solution of this application. In some other embodiments, the technical solution of this application may further detect pressure data of the user, and correspondingly, the HRV detection method, a scale questionnaire method, a behavior observation method, or the like may be used. The details are not described herein in this application.

In summary, by using the implementation method of this application, after determining that acquired pulse data includes abnormal pulse data, the electronic device calculates a ratio of an accumulated duration of the abnormal pulse data to a total duration of the pulse data, and further determines whether the ratio is greater than a first preset value. When the ratio is greater than the first preset value, the electronic device displays first physiological data of a user, where the first physiological data includes a first physiological feature parameter, thereby ensuring that the physiological data displayed to the user is correct, and improving the user experience.

The foregoing embodiments describe the solutions of the data display method provided in this application from the perspective of the hardware structure of the electronic device, and the actions performed by each software and hardware. A person skilled in the art should be easily aware that, in combination with the processing steps such as pulse data acquisition, abnormal pulse data determination, ratio calculation, and physiological data generation described in the embodiments disclosed in this specification, this application can not only be implemented by hardware or a combination of hardware and computer software. Whether a function is performed by hardware or computer software driving hardware depends on particular applications and design constraints of the technical solutions. A person skilled in the art may use different methods to implement the described functions for each particular application, but it should not be considered that the implementation goes beyond the scope of the embodiments of this application.

For example, the electronic device 100 may implement the foregoing corresponding functions in the form of functional modules. In some embodiments, the electronic device may include an acquisition module, a processing module, and a display module. The acquisition module may be configured to perform the acquisition of pulse data in any of the embodiments shown in FIG. 3 above. The display module may be configured to perform the display of physiological data in any of the embodiments shown in FIG. 3 above. The processing module may be configured to perform operations other than the acquisition of pulse data and the display of physiological data in any of the embodiments shown in FIG. 3 above. For specific content, reference may be made to the related description of the embodiments corresponding to FIG. 3, and the details are not described herein again.

It may be understood that the division of the foregoing modules is only division of logical functions. In actual implementation, the function of the acquisition module may be integrated into the sensor for implementation the function of the processing module may be integrated into the processor for implementation, and the function of the display module may be integrated into the display for implementation. As shown in FIG. 4A, the electronic device 40 includes a sensor 401, a processor 402, and a display 403. The sensor 401 may perform the acquisition of pulse data in any of the embodiments shown in FIG. 3 above. The display 403 may be configured to perform the display of physiological data in any of the embodiments shown in FIG. 3 above. The processor 402 may be configured to perform operations other than the acquisition of pulse data and the display of physiological data in any of the embodiments shown in FIG. 3 above.

For example, the sensor 401 may acquire pulse data within a first time period, a length of the first time period being a first duration. The processor 402 may be configured to: determine that the pulse data includes abnormal pulse data, the abnormal pulse data being pulse data corresponding to arrhythmia; and determine a ratio of a second duration to the first duration, the second duration being an accumulated duration of the abnormal pulse data. The display 403 may be configured to display first physiological data when the ratio is greater than a first preset value, the first preset value being a value greater than 0 and less than 1, the first physiological data including a first physiological feature parameter.

For specific content, reference may be made to the related description of the electronic device in the embodiments corresponding to FIG. 3, and the details are not described herein again.

FIG. 4A is a description of the electronic device of this application from the perspective of an independent functional entity. In another implementation scenario, each independently running functional entity may be integrated into one hardware entity. Correspondingly, as shown in FIG. 4B, in this implementation scenario, the electronic device 41 may include a processor 411, a sensor 412, and a memory 413. The memory 413 may be configured to store a program/code preinstalled by the electronic device 41, or may store code executed by the processor 411, and the like.

It should be understood that the electronic device 41 of this application may correspond to the electronic device in the embodiments corresponding to FIG. 3 of this application, where the sensor 412 is configured to perform the acquisition of pulse data in any of the embodiments shown in FIG. 3, and the processor 411 is configured to perform other processing of the electronic device other than the acquisition of pulse data in any of the embodiments shown in FIG. 3. Details are not described herein again.

For specific content, reference may be made to the related description of the electronic device in the embodiments corresponding to FIG. 3, and the details are not described herein again.

In specific implementations, corresponding to the electronic device, this application further provides a computer storage medium. The computer storage medium disposed in any device may store a program, and when the program is executed, some or all of the steps of the embodiments of the data display method provided in FIG. 3 may be performed. The storage medium in any device may be a magnetic disk, an optical disc, a read-only memory (read-only memory, ROM), a random access memory (random access memory, RAM), or the like.

In this application, the processor may be a central processing unit (central processing unit, CPU), a network processor (network processor, NP), or a combination of a CPU and an NP. The processor may further include a hardware chip. The hardware chip may be an application-specific integrated circuit (application-specific integrated circuit, ASIC), a programmable logic device (programmable logic device, PLD), or a combination thereof. The foregoing PLD may be a complex programmable logic device (complex programmable logic device, CPLD), a field-programmable gate array (field-programmable gate array, FPGA), a generic array logic (generic array logic, GAL), or any combination thereof. The memory may include a volatile memory (volatile memory), for example, a random-access memory (random-access memory, RAM). The memory may alternatively include a non-volatile memory (non-volatile memory), for example, a read-only memory (read-only memory, ROM), a flash memory (flash memory), a hard disk drive (hard disk drive, HDD) or a solid-state drive (solid-state drive, SSD). The memory may alternatively include a combination of the foregoing types of memories. The sensor may include a sensor that detects a body shape change of the user, such as an ACC sensor and a motion sensor; the sensor may alternatively include a photoelectric sensor, such as a PPG sensor, an ambient light sensor, and an ECG sensor; and the sensor may alternatively include another high-sensitivity bioelectrical sensor, such as a bio-impedance sensor.

FIG. 4B may further include a bus interface. The bus interface may include any quantity of interconnected buses and bridges. Specifically, the buses and the bridges link various circuits of one or more processors represented by the processor and a memory represented by the memory. The bus interface may further link various other circuits such as a peripheral device, a voltage stabilizer, and a power management circuit. These are known in the art. Therefore, no details are further described in this specification. The bus interface provides an interface. The sensor provides a unit for acquiring biological data of the user. The processor is responsible for bus architecture management and general processing. The memory may store messages used by the processor when the processor performs an operation.

A person skilled in the art can further understood that, the various illustrative logical blocks (illustrative logical block) and the steps (step) listed in the embodiments of this application may be implemented through electronic hardware, computer software, or a combination of the two. Whether the functions are implemented by using hardware or software depends on particular applications and a design requirement of the entire system. A person skilled in the art can use various methods to implement functions for each particular application. However, such implementations should not be considered as exceeding the protection scope of the embodiments of this application.

The various illustrative logical units and circuits described in the embodiments of this application may implement or operate the described functions by using a general purpose processor, a digital signal processor, an application-specific integrated circuit (ASIC), a field programmable gate array (FPGA) or other programmable logic devices, discrete gate or transistor logic, discrete hardware components, or a design of any combination thereof. The general purpose processor may be a microprocessor. Optionally, the general purpose processor may be any conventional processor, controller, microcontroller, or state machine. The processor may also be implemented through a combination of computing devices, for example, a combination of a digital signal processor and a microprocessor, a plurality of microprocessors, one or more microprocessors in conjunction with a digital signal processor core, or any other such configuration.

The steps of the method or algorithm described in the embodiments of this application may be directly embedded in hardware, in a software unit executed by a processor, or in a combination thereof. The software unit may be stored in a RAM, a flash memory, a ROM, an EPROM, an EEPROM, a register, a hard disk, a removable disk, a CD-ROM, or any other form of storage medium in the art. For example, the storage medium may be connected to the processor such that the processor can read information from the storage medium and write information to the storage medium. Optionally, the storage medium may be integrated in the processor. The processor and the storage medium may be disposed in the ASIC, and the ASIC may be disposed in UE. Optionally, the processor and the storage medium may be alternatively disposed in different components of the UE.

It should be understood that in various embodiments of this application, an order of sequence numbers of the foregoing processes does not indicate an execution sequence, and execution sequences of the processes should be determined according to functions and internal logics thereof and should not impose any limitation on an implementation process of the embodiments.

All or some of the foregoing embodiments may be implemented by using software, hardware, firmware, or any combination thereof. When software is used for implementation, implementation may be entirely or partially performed in the form of a computer program product. The computer program product includes one or more computer instructions. When the computer program instructions are loaded and executed on the computer, the procedure or functions according to this application are all or partially generated. The computer may be a general-purpose computer, a dedicated computer, a computer network, or other programmable apparatuses. The computer instructions may be stored in a computer-readable storage medium or may be transmitted from a computer-readable storage medium to another computer-readable storage medium. For example, the computer instructions may be transmitted from a website, computer, server, or message center to another website, computer, server, or message center in a wired (for example, a coaxial cable, an optical fiber, or a digital subscriber line (DSL)) or wireless (for example, infrared, radio, or microwave) manner. The computer-readable storage medium may be any usable medium accessible by a computer, or a message storage device, such as a server or a message center, integrating one or more usable media. The usable medium may be a magnetic medium (for example, a floppy disk, a hard disk, or a magnetic tape), an optical medium (for example, a DVD), a semiconductor medium (for example, a solid state disk (solid state disk, SSD)), or the like.

The parts of this specification are all described in a progressive manner, for same or similar parts in the embodiments, refer to such embodiments, and descriptions of each embodiment focus on a difference from other embodiments. Especially, apparatus and system embodiments are basically similar to a method embodiment, and therefore are described briefly. For related parts, reference may be made to partial descriptions in the method embodiment.

Although exemplary embodiments of this application have been described, once persons skilled in the art know the basic creative concept, they can make additional changes and modifications to these embodiments. Therefore, the following claims are intended to be construed as to cover the exemplary embodiments and all changes and modifications falling within the scope of this application.

**Claims**

1. A computer implemented data display method, comprising

   acquiring (S11) pulse data within a first time period, a length of the first time period being a first duration;
   determining (S12) that the pulse data comprises abnormal pulse data, the abnormal pulse data being pulse data corresponding to arrhythmia;
   determining (S13) a ratio of a second duration to the first duration, the second duration being an accumulated duration of the abnormal pulse data;
   displaying (S14) first physiological data when the ratio is greater than a first preset value, the first preset value being a value greater than 0 and less than 1, the first physiological data comprising a first physiological feature parameter;
   displaying the first physiological data and second physiological data when the ratio is greater than the first preset value and less than or equal to a second preset value, the second preset value being greater than the first preset value and less than 1, the second physiological data comprising a second physiological feature parameter; and
   displaying third physiological data when the ratio is less than or equal to the first preset value, the third physiological data comprising the first physiological feature parameter, the second physiological feature parameter, and a third physiological feature parameter, wherein

      the first physiological feature parameter comprises a deep sleep duration and a light sleep duration;
the second physiological feature parameter comprises a rapid eye movement duration; and

      the third physiological feature parameter comprises at least one of a deep sleep ratio, a light sleep ratio, a rapid eye movement ratio, a deep sleep continuity score, a sleep quality score, and a breathing quality score.

2. The method according to claim 1, wherein before the displaying first physiological data, the method further comprises:
   parsing the pulse data by using an acceleration ACC detection method to obtain the first physiological data.

3. The method according to claim 1, wherein before the displaying the first physiological data and second physiological data, the method further comprises:

      parsing the abnormal pulse data in the pulse data by using an acceleration ACC detection method to obtain the first physiological data; and
      parsing data in the pulse data except the abnormal pulse data by using a cardiopulmonary coupling CPC detection method to obtain the second physiological data.

4. The method according to claim 1, wherein before the displaying third physiological data, the method further comprises:

      processing the abnormal pulse data in the pulse data to obtain reconstructed pulse data; and
      parsing the reconstructed pulse data by using a CPC detection method to obtain the third physiological data.

5. The method according to claim 4, wherein the processing the abnormal pulse data in the pulse data to obtain reconstructed pulse data comprises:

      deleting the abnormal pulse data from the pulse data to obtain the reconstructed pulse data; or
      modifying the abnormal pulse data according to non-abnormal pulse data adjacent to the abnormal pulse data, to obtain the reconstructed pulse data.

6. The method according to any one of claims 1 to 5, wherein the determining that the pulse data comprises abnormal pulse data comprises:

      dividing the pulse data into k data segments, k being greater than or equal to 1, a duration corresponding to each data segment in the k data segments being a third duration, the third duration being less than the second duration;
determining whether each data segment in the k data segments satisfies a correspondence $y_t(\mathbf{w} \times \mathbf{x}_t - \mathbf{b}) \geq \mathbf{1}$, t = 1, 2, 3, ..., k, w being a normal vector of support vectors, b being a deviation value of a decision plane, $\mathbf{x}_t$ being a $t^{th}$ data segment in the k data segments, $\mathbf{y}_t$ being corresponding pulse index data indicating whether heart rhythm is abnormal; and

if $\mathbf{y}_t$ is pulse index data corresponding to arrhythmia, determining that the data segment is the abnormal pulse data when the data segment satisfies the correspondence $y_t(\mathbf{w} \times \mathbf{x}_t - \mathbf{b}) \geq \mathbf{1}$.

7. An electronic device, comprising: a sensor (401), a processor (402), and a display (403), wherein

the sensor (401) is configured to acquire (S11) pulse data within a first time period, a length of the first time period being a first duration;
the processor (402) is configured to determine (S12) that the pulse data comprises abnormal pulse data, the abnormal pulse data being pulse data corresponding to arrhythmia;
the processor (402) is further configured to determine (S13) a ratio of a second duration to the first duration, the second duration being an accumulated duration of the abnormal pulse data; and
the display (403) is configured to display (S14) first physiological data when the ratio is greater than a first preset value, the first preset value being a value greater than 0 and less than 1, the first physiological data comprising a first physiological feature parameter;
the display (403) is further configured to display the first physiological data and second physiological data when the ratio is greater than the first preset value and less than or equal to a second preset value, the second preset value being greater than the first preset value and less than 1, the second physiological data comprising a second physiological feature parameter; and
the display (403) is further configured to display third physiological data when the ratio is less than or equal to the first preset value, the third physiological data comprising the first physiological feature parameter, the second physiological feature parameter, and a third physiological feature parameter, wherein

the first physiological feature parameter comprises a deep sleep duration and a light sleep duration;
the second physiological feature parameter comprises a rapid eye movement duration; and

the third physiological feature parameter comprises at least one of a deep sleep ratio, a light sleep ratio, a rapid eye movement ratio, a deep sleep continuity score, a sleep quality score, and a breathing quality score.

8. A computer-readable storage medium, comprising a computer program, the computer program, when run on a computer, causing the computer to perform the method according to any one of claims 1 to 6.

**Patentansprüche**

1. Computerimplementiertes Datenanzeigeverfahren, umfassend:

Erfassen (S11) von Pulsdaten innerhalb eines ersten Zeitraums, wobei eine Länge des ersten Zeitraums eine erste Dauer ist;
Bestimmen (S12), dass die Pulsdaten anormale Pulsdaten umfassen, wobei die anormalen Pulsdaten Pulsdaten sind, die Arrhythmie entsprechen;
Bestimmen (S13) eines Verhältnisses von einer zweiten Dauer zu der ersten Dauer, wobei die zweite Dauer eine akkumulierte Dauer der anormalen Pulsdaten ist;
Anzeigen (S14) erster physiologischer Daten, wenn das Verhältnis größer als ein erster voreingestellter Wert ist, wobei der erste voreingestellte Wert ein Wert größer als 0 und kleiner als 1 ist, die ersten physiologischen Daten umfassend einen ersten physiologischen Merkmalsparameter;
Anzeigen der ersten physiologischen Daten und der zweiten physiologischen Daten, wenn das Verhältnis größer als der erste voreingestellte Wert und kleiner als oder gleich einem zweiten voreingestellten Wert ist, wobei der zweite voreingestellte Wert größer als der erste voreingestellte Wert und kleiner als 1 ist, die zweiten physiologischen Daten umfassend einen zweiten physiologischen Merkmalsparameter; und
Anzeigen von dritten physiologischen Daten, wenn das Verhältnis kleiner als oder gleich dem ersten voreingestellten Wert ist, die dritten physiologischen Daten umfassend den ersten physiologischen Merkmalsparameter, den zweiten physiologischen Merkmalsparameter und einen dritten physiologischen Merkmalsparameter umfassen, wobei der erste physiologische Merkmalsparameter eine Tiefschlafdauer und eine Leichtschlafdauer umfasst;
der zweite physiologischen Merkmalsparameter eine Dauer einer schnellen Augenbewegung umfasst; und
der dritte physiologische Merkmalsparameter mindestens eines von einem Tiefschlafverhältnis, einem Leichtschlafverhältnis, einem Verhältnis der schnellen Augenbewegung, einem Tiefschlafkontinuitätswert, einer Schlafqualitätsbewertung und einer Atmungsqualitätsbewertung umfasst.

2. Verfahren nach Anspruch 1, wobei vor dem Anzeigen der ersten physiologischen Daten das Verfahren ferner umfasst:
Parsen der Pulsdaten durch Verwenden eines Beschleunigungs-Erkennungsverfahrens, ACC-Erkennungsverfahrens, um die ersten physiologischen Daten zu erhalten.

3. Verfahren nach Anspruch 1, wobei vor dem Anzeigen der ersten physiologischen Daten und der zweiten physiologischen Daten das Verfahren ferner umfasst:

Parsen der anormalen Pulsdaten in den Pulsdaten durch Verwenden eines Beschleunigungs-Erkennungsverfahrens, ACC-Erkennungsverfahren, um die ersten physiologischen Daten zu erhalten; und
Parsen von Daten in den Pulsdaten mit Ausnahme der anormalen Pulsdaten durch Verwenden eines Erkennungsverfahrens einer kardiopulmonalen Kopplung, CPC-Erkennungsverfahren, um die zweiten physiologischen Daten zu erhalten.

4. Verfahren nach Anspruch 1, wobei vor dem Anzeigen der dritten physiologischen Daten das Verfahren ferner umfasst:

Verarbeiten der anormalen Pulsdaten in den Pulsdaten, um rekonstruierte Pulsdaten zu erhalten; und
Parsen der rekonstruierten Pulsdaten durch Verwenden eines CPC-Erkennungsverfahrens, um die dritten physiologischen Daten zu erhalten.

5. Verfahren nach Anspruch 4, wobei das Verarbeiten der anormalen Pulsdaten in den Pulsdaten, um rekonstruierte Pulsdaten zu erhalten, umfasst:

Löschen der anormalen Pulsdaten aus den Pulsdaten, um die rekonstruierten Pulsdaten zu erhalten; oder
Modifizieren der anormalen Pulsdaten gemäß nicht anormalen Pulsdaten angrenzend an die anormalen Pulsdaten, um die rekonstruierten Pulsdaten zu erhalten.

6. Verfahren nach einem der Ansprüche 1 bis 5, wobei das Bestimmen, dass die Pulsdaten anomale Pulsdaten umfassen, umfasst:

Teilen der Pulsdaten in k Datensegmente, wobei k größer als oder gleich 1 ist, wobei eine Dauer, die jedem Datensegment in den k Datensegmenten entspricht, eine dritte Dauer ist, wobei die dritte Dauer kleiner als die zweite Dauer ist;
Bestimmen, ob jedes Datensegment in den k Datensegmenten eine Entsprechung $y_t(w \times x_t - b) \geq 1$, t = 1, 2, 3, ..., k erfüllt, wobei w ein Normalenvektor von Stützvektoren ist, b ein Abweichungswert einer Entscheidungsebene ist, $x_t$ ein t-tes Datensegment in den k Datensegmenten ist, $y_t$ entsprechende Pulsindexdaten ist, die angeben, ob ein Herzrhythmus anormal ist; und
falls $y_t$ Pulsindexdaten ist, die Arrhythmie entsprechen, Bestimmen, dass das Datensegment die anormalen Pulsdaten ist, wenn das Datensegment die Entsprechung $y_t(w \times x_t - b) \geq 1$ erfüllt.

7. Elektronische Vorrichtung, umfassend: einen Sensor (401), einen Prozessor (402) und eine Anzeige (403), wobei

der Sensor (401) konfiguriert ist, um Pulsdaten innerhalb eines ersten Zeitraums zu erfassen (S11), wobei eine Länge des ersten Zeitraums eine erste Dauer ist;
der Prozessor (402) konfiguriert ist, um zu bestimmen (S12), dass die Pulsdaten anormale Pulsdaten umfassen, wobei die anormalen Pulsdaten Pulsdaten sind, die Arrhythmie entsprechen;
der Prozessor (402) ferner konfiguriert ist, um ein Verhältnis von einer zweiten Dauer zu der ersten Dauer zu bestimmen (S13), wobei die zweite Dauer eine akkumulierte Dauer der anormalen Pulsdaten ist; und
wobei die Anzeige (403) konfiguriert ist, um erste physiologische Daten anzuzeigen (S14), wenn das Verhältnis größer als ein erster voreingestellter Wert ist, wobei der erste voreingestellte Wert ein Wert größer als 0 und kleiner als 1 ist, wobei die ersten physiologischen Daten einen ersten physiologischen Merkmalsparameter umfassen;
wobei die Anzeige (403) ferner konfiguriert ist, um die ersten physiologischen Daten und die zweiten physiologischen Daten anzuzeigen, wenn das Verhältnis größer als der erste voreingestellte Wert und kleiner als oder gleich einem zweiten voreingestellten Wert ist, wobei der zweite voreingestellte Wert größer als der erste voreingestellte Wert und kleiner als 1 ist, die zweiten physiologischen Daten umfassend einen zweiten physiologischen Merkmalsparameter; und

die Anzeige (403) ferner konfiguriert ist, um dritte physiologische Daten anzuzeigen, wenn das Verhältnis kleiner als oder gleich dem ersten voreingestellten Wert ist, die dritten physiologischen Daten umfassend den ersten physiologischen Merkmalsparameter, den zweiten physiologischen Merkmalsparameter und einen dritten physiologischen Merkmalsparameter, wobei

der erste physiologische Merkmalsparameter eine Tiefschlafdauer und eine Leichtschlafdauer umfasst;

der zweite physiologischen Merkmalsparameter eine Dauer der schnellen Augenbewegung umfasst; und

der dritte physiologische Merkmalsparameter mindestens eines von einem Tiefschlafverhältnis, einem Leichtschlafverhältnis, einem Verhältnis der schnellen Augenbewegung, einem Tiefschlafkontinuitätswert, einer Schlafqualitätsbewertung und einer Atmungsqualitätsbewertung umfasst.

8. Computerlesbares Speichermedium, umfassend ein Computerprogramm, wobei das Computerprogramm, wenn es auf einem Computer ausgeführt wird, den Computer veranlasst, das Verfahren nach einem der Ansprüche 1 bis 6 durchzuführen.

**Revendications**

1. Procédé d'affichage de données mis en oeuvre par ordinateur, comprenant :

l'acquisition (S11) de données de pouls à l'intérieur d'une première période de temps, une longueur de la première période de temps étant une première durée ;
la détermination (S12) que les données de pouls comprennent des données de pouls anormales, les données de pouls anormales étant des données de pouls correspondant à une arythmie ;
la détermination (S13) d'un rapport d'une deuxième durée à la première durée, la deuxième durée étant une durée cumulée de données de pouls anormales ;
l'affichage (S14) de premières données physiologiques lorsque le rapport est supérieur à une première valeur prédéfinie, la première valeur prédéfinie étant une valeur supérieure à 0 et inférieure à 1, les premières données physiologiques comprenant un premier paramètre de caractéristique physiologique ;
l'affichage des premières données physiologiques et des deuxièmes données physiologiques lorsque le rapport est supérieur à la première valeur prédéfinie et inférieur ou égal à une deuxième valeur prédéfinie, la deuxième valeur prédéfinie étant supérieure à la première valeur prédéfinie et inférieure à 1, les deuxièmes données physiologiques comprenant un deuxième paramètre de caractéristique physiologique ; et
l'affichage de troisièmes données physiologiques lorsque le rapport est inférieur ou égal à la première valeur prédéfinie, les troisièmes données physiologiques comprenant le premier paramètre de caractéristique physiologique, le deuxième paramètre de caractéristique physiologique et un troisième paramètre de caractéristique physiologique, dans lequel

le premier paramètre de caractéristique physiologique comprend une durée de sommeil profond et une durée de sommeil léger ;
le deuxième paramètre de caractéristique physiologique comprend une durée de mouvement oculaire rapide ; et
le troisième paramètre de caractéristique physiologique comprend au moins l'un parmi un rapport de sommeil profond, un rapport de sommeil léger, un rapport de mouvement oculaire rapide, un score de continuité de sommeil profond, un score de qualité de sommeil et un score de qualité de respiration.

2. Procédé selon la revendication 1, dans lequel, avant l'affichage de premières données physiologiques, le procédé comprend en outre :
l'analyse des données de pouls à l'aide d'un procédé de détection d'accélération ACC afin d'obtenir les premières données physiologiques.

3. Procédé selon la revendication 1, dans lequel, avant l'affichage des premières données physiologiques et deuxièmes données physiologiques, le procédé comprend en outre :

l'analyse des données de pouls anormales dans les données de pouls à l'aide d'un procédé de détection d'accélération ACC afin d'obtenir les premières données physiologiques ; et
l'analyse de données dans les données de pouls à l'exception des données de pouls anormales à l'aide d'un procédé de détection de couplage cardiopulmonaire CPC afin d'obtenir les deuxièmes données physiologiques.

4. Procédé selon la revendication 1, dans lequel, avant l'affichage de troisièmes données physiologiques, le procédé comprend en outre :

le traitement des données de pouls anormales dans les données de pouls afin d'obtenir des données de pouls reconstruites ; et

l'analyse des données de pouls reconstruites à l'aide d'un procédé de détection CPC afin d'obtenir les troisièmes données physiologiques.

5. Procédé selon la revendication 4, dans lequel le traitement de données de pouls anormales dans les données de pouls afin d'obtenir des données de pouls reconstruites comprend :

la suppression des données de pouls anormales des données de pouls afin d'obtenir les données de pouls reconstruites ; ou la modification des données de pouls anormales en fonction des données de pouls non anormales adjacentes aux données de pouls anormales, afin d'obtenir les données de pouls reconstruites.

6. Procédé selon l'une quelconque des revendications 1 à 5, dans lequel la détermination que les données de pouls comprennent des données de pouls anormales comprend :

la division des données de pouls en k segments de données, k étant supérieur ou égal à 1, une durée correspondant à chaque segment de données dans les k segments de données étant une troisième durée, la troisième durée étant inférieure à la deuxième durée ; la détermination si chaque segment de données dans les k segments de données satisfait à une correspondance $y_t(w \times x_t - b) \geq 1$, t = 1, 2, 3,..., k, w étant un vecteur normal de vecteurs de support, b étant une valeur de déviation d'un plan de décision, $x_t$ étant le $t^{\text{ième}}$ segment de données dans les k segments de données, $y_t$ étant des données d'indice de pouls correspondantes indiquant si le rythme cardiaque est anormal ; et si $y_t$ est des données d'indice de pouls correspondant à une arythmie, la détermination que le segment de données est les données de pouls anormales lorsque le segment de données satisfait à la correspondance $y_t(w \times x_t - b) \geq 1$.

7. Dispositif électronique comprenant : un capteur (401), un processeur (402) et un afficheur (403), dans lequel

le capteur (401) est configuré pour acquérir (S11) des données de pouls à l'intérieur d'une première période de temps, une longueur de la première période de temps étant une première durée ; le processeur (402) est configuré pour déterminer (S12) que les données de pouls comprennent des données de pouls anormales, les données de pouls anormales étant des données de pouls correspondant à une arythmie ;

le processeur (402) est en outre configuré pour déterminer (S13) un rapport d'une deuxième durée à la première durée, la deuxième durée étant une durée cumulée de données de pouls anormales ; et l'afficheur (403) est configuré pour afficher (S14) des premières données physiologiques lorsque le rapport est supérieur à une première valeur prédéfinie, la première valeur prédéfinie étant une valeur supérieure à 0 et inférieure à 1, les premières données physiologiques comprenant un premier paramètre de caractéristique physiologique ; l'afficheur (403) est en outre configuré pour afficher les premières données physiologiques et deuxièmes données physiologiques lorsque le rapport est supérieur à la première valeur prédéfinie et inférieur ou égal à une deuxième valeur prédéfinie, la deuxième valeur prédéfinie étant supérieure à la première valeur prédéfinie et inférieure à 1, les deuxièmes données physiologiques comprenant un deuxième paramètre de caractéristique physiologique ; et l'afficheur (403) est en outre configuré pour afficher des troisièmes données physiologiques lorsque le rapport est inférieur ou égal à la première valeur prédéfinie, les troisièmes données physiologiques comprenant le premier paramètre de caractéristique physiologique, le deuxième paramètre de caractéristique physiologique et un troisième paramètre de caractéristique physiologique, dans lequel le premier paramètre de caractéristique physiologique comprend une durée de sommeil profond et une durée de sommeil léger ; le deuxième paramètre de caractéristique physiologique comprend une durée de mouvement oculaire rapide ; et le troisième paramètre de caractéristique physiologique comprend au moins l'un parmi un rapport de sommeil profond, un rapport de sommeil léger, un rapport de mouvement oculaire rapide, un score de continuité de sommeil profond, un score de qualité de sommeil et un score de qualité de respiration.

8. Support de stockage lisible par ordinateur, comprenant un programme informatique, le programme informatique, lorsqu'il est exécuté sur un ordinateur, amenant l'ordinateur à effectuer le procédé selon l'une quelconque des revendications 1 à 6.

Smart watch 10

FIG. 1A

**Electronic device 100**

FIG. 1B

Smart watch 10

Display screen
13

FIG. 1C

FIG. 2A

← **Sleep**                    ⋌⚬    ⋮

Day        Week        Month        Year

〈            June 21, 2018            〉
Total sleep
**8** hours and **40** minutes

● Deep sleep    ● Light sleep    ● Awake

21:45                    09:32

20:00        02:00        10:00        14:00        20:00

Total sleep
**8** hours and **40** minutes

Sleep ratio

● Deep sleep      3 hours and 20 minutes

● Light sleep      5 hours and 20 minutes

**Number of wakefulness**                    3 times

FIG. 2B

EP 3 981 326 B1

FIG. 2C

22

China Mobile  ⊞ ⁴ᴳ.ıll 🛜          👁 Ⓝ ⊙ ⁂ 87% 🔋 14:57

← **Detailed statistics**

| Day | Week | Month | Year |

<   2019-03-01   >

23:51

No abnormality Average heart rate: 68 beats/minute

200 (beats/minute)

150

100

50

00:00        06:00        12:00        18:00        00:00

\* Drag the button left and right to view more

Irregular heart rhythm ratio

● Irregular heart rhythm ratio: 4 times

○ No abnormality: 54 times

**Featured data of the day** ⓘ                    View more >

● 70 bpm  ( Automatic measurement )   2019/03/01 03:31:12 >
Irregular heart rhythm

● 65 bpm  ( Automatic measurement )   2019/03/01 01:51:13 >
No abnormality

FIG. 2D

China
Mobile ⬛ ⁴ᵍ.ᵢₗₗ 📶          👁 🔃 🕐 ⁂ 87% 🔋 14:55

← **Detailed statistics**

| Day | Week | Month | Year |

‹          2/25/2019 to 3/3/2019          ›

2019-03-02

Irregular heart rhythm ratio: 1/58

| Number of no abnormality | Number of irregular heart rhythm | Irregular heart rhythm ratio curve |

100 (times)

100%

50

50%

11.5%          8.0%  6.9%

0.0%          0.0%          1.7%

02/25  02/26  02/27  02/28  03/01  03/02  03/03

◉

* Drag the button left and right to view more

Irregular heart rhythm ratio

● Irregular heart rhythm ratio: 10 times

◉ No abnormality: 260 times

▥ You have measured heart rhythm 270 times this
week. Irregular heart rhythm is detected 10 times.
Your measurement habit is good. Keep it up!
You can click on "Go to set background resident" to
maintain the stability of device connection and
synchronize device data in time.

FIG. 2E

**10**

Acquire pulse data within a first time period — S11

Determine that the pulse data includes abnormal pulse data — S12

Determine a ratio of a second duration to the first duration — S13

Display first physiological data when the ratio is greater than a first preset value — S14

FIG. 3

**40**

Sensor — 401

Processor — 402

Display — 403

FIG. 4A

**41**

Processor — 411

Memory — 413

Bus interface

Sensor — 412

FIG. 4B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- RU 2315551 C2